Europäisches Patentamt

European Patent Office

Office européen des brevets

(11) Veröffentlichungsnummer: **0 403 935**

**A1**

# EUROPÄISCHE PATENTANMELDUNG

(21) Anmeldenummer: **90111104.7**

(22) Anmeldetag: **12.06.90**

(51) Int. Cl.5: **A61K 39/395,** //C12P21/08

(30) Priorität: **13.06.89 DE 3919294**

(43) Veröffentlichungstag der Anmeldung:
**27.12.90 Patentblatt 90/52**

(84) Benannte Vertragsstaaten:
**AT BE CH DE DK ES FR GB GR IT LI LU NL SE**

(71) Anmelder: **Max-Planck-Gesellschaft zur Förderung der Wissenschaften e.V.
Bunsenstrasse 10
D-3400 Göttingen(DE)**

(72) Erfinder: **Emmrich, Frank, Prof. Dr.
Höhenweg 4
D-8521 Braueninghof(DE)**

(74) Vertreter: **Huber, Bernhard, Dipl.-Chem. et al
Patentanwälte Dipl.-Ing.H. Weickmann,
Dipl.-Phys.Dr.K. Fincke, Dipl.-Ing.F.A.
Weickmann, Dipl.-Chem.B. Huber, Dr.-Ing.H.
Liska, Dipl.-Phys.Dr.J.Prechtel, Möhlstrasse
22
D-8000 München 80(DE)**

(54) Verwendung eines monoklonalen Antikörpers zur Behandlung einer Autoimmunerkrankung.

(57) Der monoklonale Antikörper 30F16H5 eignet sich zur Verwendung bei der Behandlung einer Autoimmunerkrankung bzw. zur Herstellung eines Arzneimittels für die Behandlung einer Autoimmunerkrankung.

EP 0 403 935 A1

**Verwendung eines monoklonalen Antikörpers zur Behandlung einer Autoimmunerkrankung**

Die Erfindung betrifft die Verwendung eines monoklonalen Antikörpers zur Behandlung einer Autoimmunerkrankung.

Autoimmunkrankheiten, auch als Autoagressionskrankheiten bezeichnet, sind durch eine Immunreaktion gegen körpereigene Substanzen verursachte Krankheiten. Beispiele für Autoimmunkrankheiten bzw. von Krankheiten, von denen man annimmt, daß sie durch eine Autoimmunreaktion verursacht werden, sind rheumatoide Arthritis, Myasthenie, Myositis, multiple Sklerose, Impfenzephalitis, Hashimoto-Thyreoiditis, phakogene Uveitis, sympathische Ophthalmie, Pemphigus, Pemphigoid, atrophische Gastritis, Colitis ulcerosa, nephrotoxische Nephritis, Postmyokardinfarkt- und Postkardiotomiesyndrom und Autoimmunzytopenien.

Die Ursache der Entstehung von Autoimmunkrankheiten ist noch nicht eindeutig geklärt. Gemäß einer Annahme entwickelt das Immunsystem Abwehrreaktionen gegen Fremdantigene, die ein Epitop tragen, welches einem körpereigenen Molekül "zum Verwechseln ähnlich" ist, so daß die dieses Fremdantigen erkennenden Rezeptoren von Lymphozyten und Antikörpern auch mit diesen Autoantigenen reagieren. Gemäß einer anderen Annahme werden Selbst-Antigene erkennende Lymphozyten im Embryonalstadium eliminiert, erst später auftretende Selbst-Antigene aber können trotzdem zu einer Immunreaktion führen. Welche Erklärung für die Ausbildung einer derartigen Erkrankung sich aber letztlich als richtig erweisen wird, jedenfalls besteht derzeit noch große Unsicherheit über den Mechanismus einer derartigen Erkrankung und insbesondere steht bis jetzt kein spezifisches Verfahren oder Arzneimittel zur zuverlässigen Behandlung einer derartigen Autoimmunerkrankung zur Verfügung. Daher werden derzeit diese Erkrankun gen meist mit unspezifischen allgemeinen Maßnahmen behandelt, wie Verabreichung antimitotischer Mittel, Unterdrückung der Immunreaktion generell, Verabreichung antiinflammatorischer Arzneimittel und ähnlichem, um die Symptome derartiger Krankheiten zu mildern oder zu bekämpfen. Diese Methoden sind jedoch generell als unbefriedigend anzusehen.

Der Erfindung liegt daher die Aufgabe zugrunde, ein spezifisch wirkendes Arzneimittel zur Behandlung von Autoimmunerkrankungen zur Verfügung zu stellen, welches nicht zu einer wesentlichen Beeinträchtigung des immunologischen Zustands des Patienten führt und keine antimitotische Wirksamkeit mit ihren unerwünschten Begleiterscheinungen aufweist.

Gelöst wird diese Aufgabe erfindungsgemäß durch die Verwendung des monoklonalen Antikörpers 30F16H5, erhältlich aus der Hybridom-Zellinie ECACC 88050502 zur Behandlung einer Autoimmunerkrankung.

Der monoklonale Antikörper 30F16H5, der erfindungsgemäß verwendet wird, wurde bereits für die Inhibierung der Infektion von Zellen durch HIV-Viren vorgeschlagen. Der erfindungsgemäß verwendete Antikörper bindet sich an das sogenannte CD4-Protein, das auf der Oberfläche von beispielsweise T-Helferlymphozyten, Makrophagen und anderen Zellen vorhanden ist.

Überraschenderweise wurde nunmehr gefunden, und hierauf beruht die Erfindung, daß dieser Antikörper auch gegen Autoimmunerkrankungen wirksam ist. So wurde beispielsweise bei Patienten mit rheumatoider Arthritis, einer typischen Autoimmunerkrankung, die über lange Zeiträume einen Blutsenkungswert von 100 und darüber aufweisen, durch Verabreichung des genannten monoklonalen Antikörpers innerhalb kurzer Zeit eine Herabsetzung auf einen Wert von etwa 20 erreicht. CD4-Protein tragende T-Zellen waren nach intravenöser Verabreichung des Antikörpers innerhalb von zwei Tagen im peripheren Blut auf weniger als 5 % reduziert und begannen erst 10 Tage nach Ende der Verabreichung peripher wieder allmählich aufzutreten.

Der Wirkmechanismus von 30F16H5 beruht auf einer sehr effektiven Entfernung (Depletion) von CD4$^+$ T-Zellen aus der Zirkulation, die nicht durch Aktivierung von Humankomplement verursacht wird. Damit stehen CD4$^+$ T-Zellen nicht mehr für den Einstrom in das Entzündungsgewebe zur Verfügung. 30F16H5 ist daher zur kurzfristigen Induktion einer Helferzellsuppression bei der Behandlung von Autoimmunkrankheiten oder zur Verhinderung von Transplantatrejektionen geeignet. Außerdem ist zu erwarten, daß der erfindungsgemäß verwendete Antikörper zur Erzeugung einer Immuntoleranz gegen bestimmte Antigene geeignet ist. Aus Nature 320: 449, 1986 ist es bekannt, im Tiermodell durch kurzfristige (14-tägige) anti-CD4 Behandlung mit depletierenden Antikörpern eine Phase zu erzeugen, in der gegen Antigene, die innerhalb dieser 14 Tage appliziert werden, eine Immuntoleranz induziert werden kann. Nach Absetzen der anti-CD4 Behandlung war keine Immunantwort mehr gegen das verwendete Antigen auslösbar. Dies sollte mit dem Antikörper 30F16H5 erfindungsgemäß für therapeutische Zwecke in der Humanmedizin nutzbar sein.

Die Gewinnung des monoklonalen Antikörpers aus der Hybridom-Zellinie erfolgt nach an sich bekannten Methoden. Die Zellen werden kultiviert und die monoklonalen Antikörper z.B. aus dem Zellüberstand durch Protein A Sepharose-Chromatographie erhalten.

Ein weiterer Gegenstand der Erfindung ist die Verwendung des monoklonalen Antikörpers 30F16H5 zur Herstellung eines Arzneimittels für die Behandlung einer Autoimmunerkrankung. Hierbei können weitere übliche pharmazeutische Träger- und Zusatzstoffe eingeschlossen sein.

Zur Herstellung dieses Arzneimittels bringt man den monoklonalen Antikörper 30F16H5, gegebenenfalls mit üblichen Träger-und Zusatzstoffen in eine pharmazeutisch geeignete Anwendungsform.

Geeignete Anwendungsformen für das erfindungsgemäße Arzneimittel sind bevorzugt Lösungen, die zur intravenösen Injektion geeignet sind.

Die folgenden Beispiele erläutern die Erfindung.

**Beispiel 1**

Immunisierung und Produktion der Hybridome zur Herstellung des monoklonalen Antikörpers 30F16H5:

$4 \times 10^6$ bis $8 \times 10^6$ Zellen des menschlichen CD4-positiven Helfer-T-Lymphozyten Klons 2C11 (Emmrich & Kaufmann, Infection and Immunity 51 (1986) 879) wurden dreimal jeweils im Abstand von 7 Tagen in weibliche BALB/c-Mäuse intraperitoneal injiziert. Es erfolgte eine weitere Immunisierung nach drei Wochen. Am darauffolgenden Tage wurde die gleiche Zellmenge intravenös verabfolgt. 3 Tage nach der letzten Injektion wurden die Tiere getötet und die Milzzellen nach der Methode von Köhler und Milstein (Nature 256 (1975) 495) mit X63.Ag8.653 Myelomazellen fusioniert (Kearney et al. J. Immunol. 123 (1979) 1548).

Nach 10 bis 14 Tagen wurde von den wachsenden Hybridomen Kulturüberstand entnommen und deren Antikörperaktivität in funktionellen Tests in vitro untersucht. Der Isotyp des Antikörpers 30F16H5 wurde mittels ELISA (Emmrich et al., Eur. J. Immunol. 13 (1983) 273) als Maus-IgG1 ermittelt. Eine Probe der Hybridomzellen wurde bei ECACC unter der Eingangsnummer 88050502 hinterlegt.

Die Spezifität des Antikörpers 30F16H5 wurde methodisch durch cell-ELISA, APAAP-Färbung und Zytofluorometrie nachgewiesen. In Tabelle 1 sind repräsentative Ergebnisse des Einsatzes von 1 µg/ml 30F16H5 bei der Bindung an drei verschiedene menschliche Zellpopulationen dargestellt. Eine lymphoblastoide B-Zellinie wurde durch Transformation von menschlichen B-Lymphozyten (Steinitz et al., Immunobiology 156 (1979) 41) erzeugt und als homogene B-Zellpopulation eingesetzt. CD4-und CD8-T-Zellen wurden über ein mehrstufiges Verfahren aus menschlichem Blut isoliert (Emmrich et al., Proc. Natl. Acad. Sci. USA 83 (1986) 8298). Hierfür wurde zunächst ein Ficolldichtegradient zur Trennung der mononukleären Zellen verwendet. Adhärente Zellen wurden an Plastikoberflächen depletiert, sodann wurden T-Lymphozyten durch Rosettierung mit stabilisierten Schafserythrozyten gewonnen. Nach Lysierung der Erythrozyten wurden die T-Zellen mit kommerziell erhältlichen anti-CD4 und anti-CD8 Antikörpern markiert und als Antikörper negative Population mit einem Fluoreszenzaktivierten Zellsortiergerät getrennt. Die Kontamination mit anderen Zellen lag für beide Populationen unter 1%. Die Markierung mit Antikörpern erfolgte nach einschlägigen Methoden (Gathings et al., Eur. J. Immunol. 7 (1977) 804) unter Verwendung eines anti-Maus-Immunoglobulin A Antikörpers, der mit FITC (Fluoresceinisothiocyanat) gekoppelt war.

Tabelle 1 zeigt, daß nur CD4-T-Zellen durch den Antikörper 30F16H5 erkannt werden. Da keine weiteren populationsdifferenten Antigene außer CD4 und CD8 auf den so charakterisierten Zellen bekannt sind, ist die Folgerung schlüssig, daß 30F16H5 das CD4-Antigen erkennt. Dies wurde durch Radioimmunpräzipitationsexperiment bestätigt.

3

Tabelle 1

|  | % fluoreszenzpositive Zellen | | mittlere Fluoreszenzintensität |
|---|---|---|---|
| Zellpopulation | -1) | 16H5 2) | 16H5 2) |
| LBL 3) | 2,3 | 3,4 | 48 |
| CD8-T-Zellen | 3,2 | 3,5 | 50 |
| CD4-T-Zellen | 3,8 | 84,6 | 658 |

1) Kontrolle bei alleinigem Einsatz eines FITC-markierten Zweitantikörpers (Ziege anti Maus-Ig mit Fluorescein)

2) Antikörper 30F16H5 und Zweitantikörper

3) Lymphoblastoide B-Zellinie

**Beispiel 2**

Die nach Beispiel 1 erhaltene 30F16H5-Präparation wurde auf biologische Sicherheit geprüft. Sie war nicht toxisch, enthält kein Virusantigen (getestet im MAP-Test für Hantavirus. Lymphocytic choriomeningitis virus (LCM), reovirus type 3 (reo 3). Sendai virus, Polyomavirus. Ectromelia virus, Mouse rotavirus (EDIM), K virus (K), Minute virus of mice (MVM), Mouse adenovirus, Theiler's encephalomyelitis virus (TEMV), Mouse hepatitis virus (MHV), Pneumonia virus of mice (PVM), Mouse cytomegalovirus (MCMV), Mycoplasma pulmonis, Thymic virus) und wurde ohne Nebenwirkungen bei Primaten appliziert.

Einer 54-jährigen Frau mit schwerer Rheumatoidarthritis wurden 7 Tage jeweils 20 mg affinitätschromatografisch gereinigter Antikörper 30F16H5 infundiert. Unter dieser Therapie konnten nach 3 Tagen keine $CD4^+$-T-Zellen mehr in der Zirkulation nachgewiesen werden, die Entzündungsmerkmale verbesserten sich dramatisch (Abnahme der Blutsenkungsgeschw. von 100 auf 23 in der ersten Stunde, Abnahme des CRP von 100 auf 16). Danach ist der Antikörper 30F16H5 wesentlich besser für die $CD4^+$-T-Zelldepletion geeignet als alle bisher zu diesem Zweck eingesetzten Antikörper (Herzog et al., Lancet, 1987: 1461). Nebenwirkungen wurden nicht beobachtet.

**Ansprüche**

1. Verwendung des monoklonalen Antikörpers 30F16H5, erhältlich aus der Hybridom-Zellinie ECACC 88050502 zur Behandlung einer Autoimmunerkrankung.

2. Verwendung des monoklonalen Antikörpers 30F16H5, erhältlich aus der Hybridom-Zellinie ECACC 88050502 zur Herstellung eines Arzneimittels für die Behandlung einer Autoimmunerkrankung.

3. Verwendung des Antikörpers gemäß Anspruch 1 oder 2 zusammen mit üblichen pharmazeutischen Träger- und Zusatzstoffen für den Zweck von Anspruch 1 oder 2.

4

Europäisches
Patentamt

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

EP 90 11 1104

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,P | WO-A-90 02 199 (MAX PLANCK-GESELL-SCHAFT ZUR FÖRDERUNG DER WISSEN-SCHAFTEN, E.V.)  \* Die Ansprüche \* | 2,3 | A 61 K 39/395// C 12 P 21/08 |
| D,Y | THE LANCET, 19. Dezember 1987, Seiten 1461-1462, London, GB; C. HERZOG et al.: "Monoclonal anti-CD4 in arthritis"  \* Das ganze Dokument \* | 2,3 | |
| A | US-A-4 695 459 (L. STEINMAN et al.)  \* Das ganze Dokument \* | 2,3 | |

**RECHERCHIERTE SACHGEBIETE (Int. Cl.4)**

C 12 P
C 07 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: 2,3

Unvollständig recherchierte Patentansprüche:

Nicht recherchierte Patentansprüche: 1

Grund für die Beschränkung der Recherche:

Verfahren zur chirurgischen oder therapeutischen
Behandlung des menschlichen oder tierischen
Körpers (Siehe Art. 52(4) des Europäischen
Patentübereinkommens)

| Recherchenort | Abschlußdatum der Recherche | Prüfer |
|---|---|---|
| DEN HAAG | 02-10-1990 | NOOIJ |